(19) Europäisches Patentamt
European Patent Office
Office européen des brevets

(11) EP 2 621 494 B1

(12) EUROPEAN PATENT SPECIFICATION

(45) Date of publication and mention of the grant of the patent:
**07.11.2018 Bulletin 2018/45**

(21) Application number: **11767661.9**

(22) Date of filing: **16.09.2011**

(51) Int Cl.:
***A61K 31/439*** *(2006.01)* ***A61K 9/00*** *(2006.01)*

(86) International application number:
**PCT/EP2011/066062**

(87) International publication number:
**WO 2012/041717 (05.04.2012 Gazette 2012/14)**

(54) **USE OF MAGNESIUM STEARATE IN DRY POWDER FORMULATIONS FOR INHALATION**

VERWENDUNG VON MAGNESIUMSTEARAT IN TROCKENPULVERFOMULIERUNGEN ZUR INHALATION

UTILISATION DE STÉARATE DE MAGNÉSIUM DANS DES PRÉPARATIONS EN POUDRE SÈCHE DESTINÉES À ÊTRE INHALÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.09.2010 EP 10183018**

(43) Date of publication of application:
**07.08.2013 Bulletin 2013/32**

(73) Proprietor: **Chiesi Farmaceutici S.p.A.**
**43100 Parma (IT)**

(72) Inventors:
- **COCCONI, Daniela**
  **I-43100 Parma (IT)**
- **DAGLI ALBERI, Massimiliano**
  **I-43100 Parma (IT)**
- **BUSCA, Andrea**
  **I-43100 Parma (IT)**
- **SCHIARETTI, Francesca**
  **I-43100 Parma (IT)**

(74) Representative: **Minoja, Fabrizio**
**Bianchetti Bracco Minoja S.r.l.**
**Via Plinio, 63**
**20129 Milano (IT)**

(56) References cited:
**WO-A1-00/28979**
**WO-A1-2005/004845**
**WO-A1-2010/015324**
**US-B1- 6 528 096**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 621 494 B1

## Description

### TECHNICAL FIELD

The present invention concerns carrier particles for use in dry powder formulations for inhalation, and formulations thereof.

In particular the present invention relates to the use of magnesium stearate in powder formulations for inhalation comprising carrier particles of alpha-lactose monohydrate to inhibit or reduce chemical degradation of an active ingredient bearing a group susceptible to hydrolysis.

### BACKGROUND OF THE INVENTION

Dry powder inhalation (DPI) drug therapy has been used for many years to treat respiratory conditions such as asthma, chronic obstructive pulmonary disease (COPD), and allergic rhinitis.

Compared to oral drug intake, only relatively small doses are needed for effective therapy as first pass metabolism is significantly reduced. Such small doses reduce the body's exposure to the drug and minimise side effects. Systemic adverse effects are also reduced as topical lung delivery takes the drug directly to the site of action. Lower dosage regimens may also provide considerable cost savings, particularly where expensive therapeutic agents are concerned.

Dry powder forms are typically formulated by mixing the drug in micronised form with physiologically acceptable pharmacologically inert coarse carrier particles, giving rise to ordered mixture where the micronised active particles adhere to the surface of the carrier particles whilst in the inhaler device.

During inhalation, the drug particles separate from the surface of carrier particles and penetrate into the lower lungs, while the larger carrier particles are mostly deposited in the oropharyngeal cavity.

To promote the release of the drug particles from the surface carrier particles and, hence, to increase the fraction of respirable particles, additives with lubricant or anti-adherent properties have been proposed in the art.

A particularly useful additive has been found to be magnesium stearate.

The benefit of using magnesium stearate in dry powders is taught for example in U.S. Pat. No. 6,528,096, which specifically teaches that magnesium stearate can be used to alter the surface properties of carrier particles and thereby improve the properties of dry powder formulations. This reference also reports an "advantageous relationship" between surface coating carrier particles with magnesium stearate and the fine particle fraction (respirable fraction) of the emitted dose.

Besides the delivered respirable fraction, another important requirement is that the active ingredient should be chemically stable in the dry powder pharmaceutical formulations on storage. In fact, it is known that active substances could demonstrate instability to one or more factors, i.e. heat, light or moisture, and various precautions must be taken in formulating and storing such substances to ensure that the pharmaceutical products remain in an acceptable condition for use over a reasonable period of time, such that they have an adequate shelf-life.

In particular, active ingredients bearing certain groups such as carbonate, carbamate and ester, in the presence of high temperature and/or percentage of moisture could give rise to degradation products occurring through hydrolysis pathway.

The present inventors have found that, in the presence of magnesium stearate, active ingredients bearing chemical groups prone to hydrolysis are more chemically stable upon storage in particular stressing conditions i.e., in the presence of high temperature and/or high percentage of moisture.

WO 00/28979 describes the use of magnesium stearate in dry powder formulations for inhalation to improve resistance to moisture and to reduce the effect of penetrating moisture on the fine particle fraction, i.e. the respirable fraction of an inhaled formulation.

Such interference with physical interactions between a carrier and a drug substance is distinct from chemical instability resulting from degradation.

WO 2005/004845 discloses the use of magnesium stearate to inhibit or reduce chemical interaction between an active ingredient substance and a carrier in a solid pharmaceutical formulation, wherein the active ingredient substance is susceptible to chemical interaction with the carrier through Maillard reaction.

Said reaction involves the formation of adducts between amines and reducing sugars such as lactose. Thus it is distinct from hydrolysis and concern drugs active ingredients bearing primary or secondary amino groups.

### SUMMARY OF THE INVENTION

The present invention is directed to the use of magnesium stearate in powder formulations for inhalation comprising carrier particles of alpha-lactose monohydrate and an active ingredient bearing a group susceptible to hydrolysis

selected from the group consisting of carbonate, carbamate and ester, to inhibit or reduce chemical degradation of said active ingredient, wherein the carrier particles have the mass diameter comprised between 80 and 500 micron and wherein magnesium stearate is present in an amount comprised between 0.05 and 1.5% based on the total weight of the carrier and coats the surface of the carrier particles to an extent equal to or higher than 60%.

The chemical stability of the active ingredient in the powder formulation may thereby be improved.

**DEFINITIONS**

The terms "active drug", "active ingredient", "active" and "active substance", and "active compound" are used as synonymous.

The terms "muscarinic receptor antagonists", "antimuscarinic drugs" and "anticholinergic drugs" are used as synonymous.

The term 'coating' refers to the covering of the surface of the carrier particles by forming a (mono)molecluar film of magnesium stearate around said particles as reported in the sketch of Figure 1.

The percentage of surface coating indicate the extent by which magnesum stearate coats the surface of all the carrier particles.

The expression 'to inhibit or reduce chemical degradation of an active ingredient' means that, upon storage, degradation product(s) arising from hydrolysis of a group susceptible to hydrolytic cleavage are not formed or are formed in a lesser amount than the formulation comprising no magnesium stearate.

"Single dose" refers to the quantity of active ingredient administered at one time by inhalation upon actuation of the inhaler.

"Actuation" refers to the release of active ingredient from the device by a single activation (e.g. mechanical or breath).

The expression "chemically stable" refers to a formulation that meets the requirements of the ICH Guideline Q1A referring to "Stability Testing of new Active Substances (and Medicinal Products)".

In general terms, the particle size of particles is quantified by measuring a characteristic equivalent sphere diameter, known as volume diameter, by laser diffraction.

The particle size can also be quantified by measuring the mass diameter by means of suitable known instruments, such as sieving.

The volume diameter (VD) is related to the mass diameter (MD) by the density of the particles (assuming a size independent density for the particles).

In the present application, the particle size interval is expressed in terms of mass diameter. The particle size distribution is generally expressed in terms of: i) the volume median diameter (VMD) or the mass median diameter (MMD) which corresponds to the diameter of 50 percent of the particles by volume or weight respectively, e.g d(0.5), and ii) the mass or volume diameter of 10% and 90% of the particles, respectively, e.g d(0.1) and d(0.9).

The expression 'respirable fraction' refers to an index of the percentage of active particles which would reach the deep lungs in a patient.

The respirable fraction, also termed fine particle fraction, is commonly evaluated using suitable *in vitro* apparata such as Multistage Cascade Impactor or Multi Stage Liquid Impinger (MLSI) according to procedures reported in common Pharmacopoeias.

**FIGURES**

**Figure 1 -** Sketch of the film forming process around a single carrier particles.
**Figure 2** - Plot of CHF 5551.02 content versus time (ambient = 60% relative humidity, R.H.).

**DETAILED DESCRIPTION OF THE INVENTION**

The invention finds application in dry powder formulations for inhalation comprising carrier particles of alpha-lactose monohydrate and at least one micronized active ingredient.

Advantageously, said coarse carrier particles have a mass diameter (MD) between 80 and 500 micron.

In certain embodiments of the invention, the MD is comprised between 90 and 150 micron.

In other embodiments, the MD is comprised between 150 and 400 micron, and preferably between 210 and 355 micron.

The desired particle size may be obtained by sieving.

When their MD is comprised between 150 and 400 micron, the coarse carrier particles have preferably a

relatively highly fissured surface, that is, on which there are clefts and valleys and other recessed regions, referred to herein collectively as fissures.

The "relatively highly fissured" coarse particles can be defined in terms of fissure index or rugosity coefficient as described in WO 01/78695 and WO 01/78693, and they can be characterized according to the description therein reported.

Said coarse carrier particles may also be characterized in terms of tapped density or total intrusion volume measured as reported in WO 01/78695.

The tapped density of the coarse carrier particles is advantageously less than 0.8 g/cm$^3$, preferably between 0.8 and 0.5 g/cm$^3$.

The total intrusion volume is of at least 0.8 cm$^3$ preferably at least 0.9 cm$^3$.

The formulation may also comprise fine particles of a physiologically acceptable pharmacologically- inert material with a mass median diameter (MMD) equal to or less than 10 micron.

The percentage of said fine particles is advantageously comprised between 0.1 and 20% of the total amount of the formulation, preferably between 5 and 15%.

Preferably, the coarse particles and the fine particles are constituted of the same physiologically acceptable pharmacologically- inert material.

The amount of magnesium stearate present in the formulation varies depending on both the dry powder inhaler and type of the active ingredient. The skilled person, aware of the physical and chemical properties of the active ingredient and of the type of inhaler, for example single dose or multidose, will be able to select an appropriate amount.

Advantageously, the amount of magnesium stearate may be comprised between 0.05 and 1.5% by weight of the carrier. More advantageously it may be comprised between 0.1 and 1.0%.

In a preferred embodiment, it may be between 0.15 and 0.5%, more preferably between 0.2 and 0.4% w/w, while in other preferred embodiments it may be between 0.5 and 1.5% by weight or between 0.8 and 1.0% w/w.

The carrier particles are subjected to coating with magnesium stearate particles.

By applying the conditions disclosed in the co-pending application EP 10158951.3, an extent of coating of more than 60%, advantageously higher than 70%, more advantageously of at least 80%, preferably higher than 85%, more preferably higher than 90%, even more preferably higher than 95% could be obtained.

The extent to which magnesium stearate coats the surface of the carrier particles of alpha-lactose monohydrate may be determined by first measuring the water contact angle, and then applying the equation known in the literature as Cassie or Cassie and Baxter, reported anyway as follows (Colombo I et al Il Farmaco 1984, 39(10), p. 338):

$$\cos\vartheta_{mixture} = f_{MgSt}\cos\vartheta_{MgSt} + f_{lactose}\cos\vartheta_{lactose}$$

where $f_{MgSt}$ and $f_{lactore}$ are the surface area fractions of magnesium stearate and of lactose;
$\vartheta_{MgSt}$ is the water contact angle of magnesium stearate;
$\vartheta_{lactose}$ is the water contact angle of lactose
$\vartheta_{mixture}$ are the experimental contact angle values.

The measure of the contact angle between a liquid and a solid surface is commonly used in the art for determining the wettability of solids. This approach is based on the capability of a liquid to spread spontaneously over the surface of a solid to reach a thermodynamic equilibrium.

For the purpose of the invention, the contact angle may be determined with methods that are essentially based on goniometric measurement. These imply the direct observation of the angle formed between the solid substrate and the liquid under testing. It is therefore quite simple to carry out, being the only limitation related to possible bias stemming from intra-operator variability. It should be, however, underlined that this drawback can be overcome by adoption of a fully automated procedure, such as a computer assisted image analysis.

A particularly useful approach is the sessile or static drop method which is typically carried out by depositing a liquid drop onto the surface of the powder in form of disc obtained by compaction (known as compressed powder disc method).

Typically, the procedure is carried out as follows:

The compressed disc is prepared by adding the sample into the die of a press and a compression force of 5 kN is applied for 3 minutes. Then the compressed disc is placed on a plate of a surface wettability tester and a water drop of about 10 μl is formed on the surface of the disc.

A suitable surface wettability tester is, for example, that available from Lorentzen & Wettre GmbH.

The pictures are taken with a videocamera and the water contact angles values are given by a computer assisting in the analysis of the image.

If a fully automated procedure is not available, the base (b) and the height (h) of the drop are measured on the display using a mobile reading scale, then the water contact angles (WCA) are calculated by applying the following formula:

$$WCA = [\text{arctg } 2h/b] x 2 x 180/\pi$$

Typically the values are calculated as a mean of three different measurements taken at room temperature. The precision is usually of about ± 5°.

Advantageously, by using the sessile drop method, and considering as reference water contact values of 12° for alpha-lactose monohydrate and of 118° for magnesium stearate, the experimental water contact angle is at least of 34°, more advantageously equal to or higher than 36°, preferably equal to or higher than 39°, more preferably equal to or higher than 50°.

By applying the conditions disclosed in the co-pending application EP 10158951.3, experimental water contact angles equal to or higher than 90°, preferably higher than 100° could be obtained.

The extent to which the magnesium stearate coats the surface of the lactose particles may also be determined by scanning electron microscopy (SEM), a versatile analytical technique well known in the art.

Such microscopy may be equipped with an EDX analyzer (an Electron Dispersive X- ray analyzer), that can produce an image selective to certain types of atoms, for example magnesium atoms. In this manner it is possible to obtain a clear data set on the distribution of magnesium stearate on the surface of carrier particles.

SEM may alternatively be combined with IR or Raman spectroscopy for determining the extent of coating, according to well known procedures.

Another analytical technique that may advantageously be used is X-ray photoelectron spectroscopy (XPS), by which it has been possible to calculate both the extent of coating and the depth of the magnesium sterate film around the lactose particles.

XPS measurements may be taken with commercially available instruments such as Axis-Ultra instrument from Kratos Analytical (Manchester UK), typically using monochromated Al Kα radiation according to known procedures.

The active ingredient is a drug for inhalation bearing a chemical group susceptible to hydrolysis such as carbonate, carbamate or ester, preferably a carbonate or a carbamate, more preferably a carbonate group.

Drugs bearing said groups typically behave as soft drugs and, once inhaled, are degraded by hydrolysis to inactive compounds which are rid of any systemic side effects.

They normally belong to classes which could exhibit undesired side effects due to systemic absorption such as muscarinic receptor antagonists, phosphodiesterase-4 inhibitors, and steroids for inhalation.

Thus, for example, the active ingredient may contain the group (I)

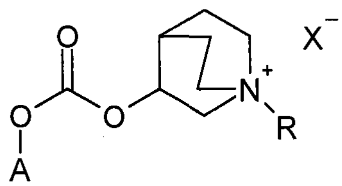

**(I)**

such as the antimuscarinic drugs disclosed in WO 2010/015324 wherein:
A may be an optionally substituted aryl or heteroaryl or arylalkyl or heteroarylalkyl or a group of formula (a)

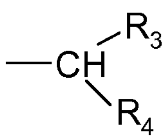

(a)

wherein

$R_3$ and $R_4$ are the same or different and may be independently selected from the group comprising H, ($C_3$-$C_8$)-cycloalkyl, aryl or heteroaryl, wherein said aryl or heteroaryl may be optionally substituted with a halogen atom or with one or several substituents independently selected from the group consisting of OH, O-($C_1$-$C_{10}$)-alkyl, oxo (=O), SH, S-($C_1$-$C_{10}$)-alkyl, $NO_2$, CN, $CONH_2$, COOH, ($C_1$-$C_{10}$)-alkoxycarbonyl, ($C_1$-$C_{10}$)-alkylsulfanyl, ($C_1$-$C_{10}$)-alkylsulfinyl, ($C_1$-$C_{10}$)-alkylsulfonyl, ($C_1$-$C_{10}$)-alkyl and ($C_1$-$C_{10}$)-alkoxyl or when $R_3$ and $R_4$ are both independently aryl or heteroaryl they may be linked through a Y group which may be a $(CH_2)_n$ with n =0-2, wherein when n=0 Y is a single bond, forming a tricyclic ring system wherein the carbon atom of $(CH_2)_n$ may be substituted by a heteroatom selected from O, S, N and with the proviso that $R_3$ and $R_4$ are not both H simultaneously;
and R is a residue selected from:

- ($C_1$-$C_{10}$)-alkyl, ($C_2$-$C_{10}$)-alkenyl and ($C_2$-$C_{10}$)-alkynyl optionally substituted with a group selected from:
- a group selected from of $NH_2$, $NR_1R_2$, $CONR_1R_2$, $NR_2COR_1$, OH, $SOR_1$, $SO_2R_1$, SH, CN, $NO_2$ and alicyclic compounds;
- Z-$R_1$, wherein Z is selected from CO, O, COO, OCO, $SO_2$, S, SO, COS and SCO or it is a bond and
- ($C_3$-$C_8$)-cycloalkyl.

$R_1$ is a residue selected from:

- alicyclic compound optionally substituted with one or several substituents independently selected from OH, oxo (=O), SH, $NO_2$, CN, $CONH_2$, $NR_2CO$-($C_1$-$C_x$)-alkyl, COOH, ($C_1$-$C_{10}$)-alkoxycarbonyl, ($C_1$-$C_{10}$)-alkylsulfanyl, ($C_1$-$C_{10}$)-alkylsulfinyl, ($C_1$-$C_{10}$)-alkylsulfonyl, ($C_1$-$C_{10}$)-alkyl and ($C_1$-$C_{10}$)-alkoxyl $NR_2CO$-($C_1$-$C_{10}$)-alkyl;
- aryl optionally substituted with $NR_2CO$-($C_1$-$C_{10}$)-alkyl, ($C_1$-$C_{10}$)-alkyl, O-($C_1$-$C_{10}$)-alkyl or halogen and
- heteroaryl optionally substituted with $NR_2CO$-($C_1$-$C_{10}$)-alkyl or halogen.

$R_2$ is a group selected from H, phenoxycarbonyl, benzyloxycarbonyl, ($C_1$-$C_{10}$)-alkoxycarbonyl, ($C_1$-$C_{10}$)-alkylcarbonyl, ($C_1$-$C_{10}$)-alkylsulfonyl and ($C_1$-$C_{10}$)-alkyl.
$X^-$ is a physiologically acceptable anion such as bromide, chloride and trifluoroacetate, preferably chloride.

A preferred group of compounds of formula (I) is that wherein R is a methyl substituted by -Z-$R_1$ group wherein Z is CO and $R_1$ is thienyl, according to formula **(II):**

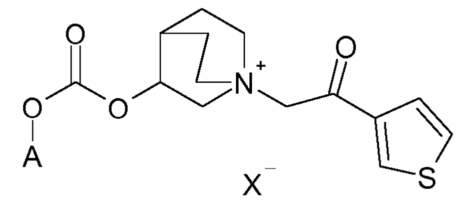

**(II)**

A more preferred group of compounds of formula (II) is that wherein A is a group of formula (a) wherein $R_3$ and $R_4$ are both phenyl, optionally substituted with one or more halogen atoms.

Otherwise the active ingredient may contain the group

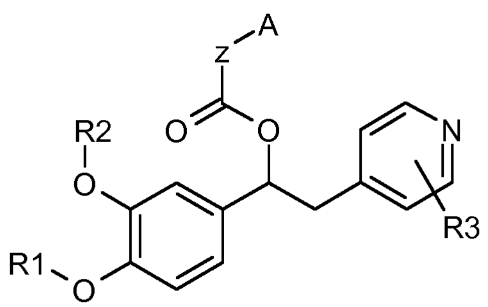

**(III)**

such as carbonate and carbamate phosphodiesterase-4 inhibitors disclosed in the co-pending application n. WO 2009/018909 wherein

Z is selected from the group consisting of
$O(CH_2)_p$ wherein p=0, 1, 2 or 3 or $NR_6$ wherein $R_6$ is H or a linear or branched ($C_1$-$C_4$) alkyl;
$R_1$ and $R_2$ are different or the same and are independently selected from the group consisting of

- H;
- linear or branched ($C_1$-$C_6$) alkyl, optionally substituted by one or more substituents selected from ($C_3$-$C_7$) cycloalkyl or ($C_5$-$C_7$) cycloalkenyl;
- ($C_3$-$C_7$) cycloalkyl;
- ($C_5$-$C_7$) cycloalkenyl;
- linear or branched ($C_2$-$C_6$) alkenyl; and
- linear or branched ($C_2$-$C_6$) alkynyl.

$R_3$ is one or more substituents independently selected from the group consisting of H, CN, $NO_2$, $CF_3$ and halogen atoms.

A is a ring system, that is a mono- or bicyclic ring which may be saturated, partially unsaturated or unsaturated, such as aryl, ($C_3$-$C_8$) cycloalkyl or heteroaryl, said ring system A having 5 to 10 ring atoms in which at least one ring atom is a heteroatom (e.g. N, S or O), in which the optional substituent $R_x$ on the A ring system may be one or more, may be the same or different, and is independently selected from the group consisting of:

- linear or branched ($C_1$-$C_6$) alkyl optionally substituted by one or more ($C_3$-$C_7$) cycloalkyl;
- linear or branched ($C_2$-$C_6$) alkenyl optionally substituted by one or more ($C_3$-$C_7$) cycloalkyl;
- linear or branched ($C_2$-$C_6$) alkynyl optionally substituted by one or more ($C_3$-$C_7$) cycloalkyl;
- ($C_5$-$C_7$) cycloalkenyl;
- phenyl;
- ($C_3$-$C_7$) heterocycloalkyl;
- $OR_7$ wherein $R_7$ is selected from the group consisting of
- H;
- ($C_1$-$C_{10}$) alkyl optionally substituted by one or more ($C_3$-$C_7$) cycloalkyl;
- ($C_3$-$C_7$) cycloalkyl;
- ($C_1$-$C_4$) alkyl-($C_3$-$C_7$) heterocycloalkyl;
- CO ($C_1$-$C_6$) alkyl;
- COO ($C_1$-$C_6$) alkyl;
- phenyl;
- benzyl;
- ($C_1$-$C_{10}$) alkyl-$NR_8R_9$ wherein $R_8$ and $R_9$ are independently selected from the group consisting of H, linear or branched ($C_1$-$C_6$) alkyl and they form with the nitrogen atom to which they are linked a saturated, partially saturated or unsaturated ring, preferably $NR_8R_9$ is linked to ($C_1$-$C_{10}$) alkyl forming for example saturated, partially saturated or unsaturated piperidine, oxazine, imidazole rings, wherein these rings are optionally substituted by ($C_1$-$C_4$) alkyl; and
- halogen atoms;
- CN;
- $NO_2$;
- $NR_{10}R_{11}$ wherein $R_{10}$ and $R_{11}$ are different or the same and are independently selected from the group consisting of
- H;
- linear or branched ($C_1$-$C_6$) alkyl, optionally substituted with phenyl or ($C_3$-$C_7$) cycloalkyl;
- $COC_6H_5$;
- CO-($C_1$-$C_4$) alkyl;
- COO-($C_1$-$C_4$) alkyl;
- CONH-($C_1$-$C_6$) alkyl-$R_{12}$, wherein $R_{12}$ is selected from the group consisting of
- H;
- ($C_1$-$C_4$) alkyl;
- $OR_4R_5$; and
- CONH ($C_1$-$C_4$) alkyl-N($C_1$-$C_4$) alkyl;

  or they form with the nitrogen atom to which they are linked a saturated or partially saturated ring, preferably a

piperidyl ring;

- $(C_1-C_4)$ alkyl-$NR_{10}R_{11}$;
- $COR_{12}$ wherein $R_{12}$ is phenyl or linear or branched $(C_1-C_6)$ alkyl;
- oxo;
- $HNSO_2R_{13}$ wherein $R_{13}$ is $(C_1-C_4)$ alkyl or a phenyl optionally substituted with halogen atoms or with a $(C_1-C_4)$ alkyl group;
- $SO_2R_{14}$ wherein $R_{14}$ is $(C_1-C_4)$ alkyl, OH or $NR_{10}R_{11}$ wherein $R_{10}$ and $R_{11}$ are as defined above;
- $SOR_{15}$ wherein $R_{15}$ is phenyl or $(C_1-C_4)$ alkyl;
- $SR_{16}$ wherein $R_{16}$ is H, phenyl or $(C_1-C_4)$ alkyl;
- $COOR_{17}$ wherein $R_{17}$ is H, $(C_1-C_4)$ alkyl, phenyl or benzyl; and
- $(CH_2)_qOR_{18}$, wherein q=1, 2, 3 or 4 and $R_{18}$ is H or $(C_1-C_4)$ cycloalkyl,

and pharmaceutically acceptable salts and N-oxides on the pyridine ring thereof.

The active ingredient may also be a steroid for inhalation bearing an ester group of formula (IV)

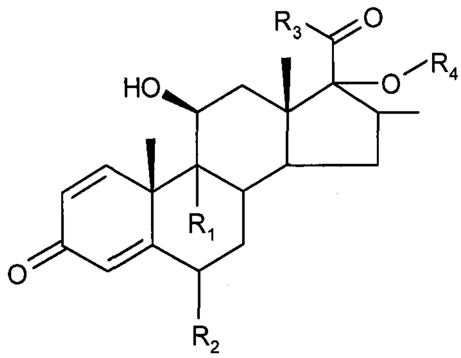

**(IV)**

wherein $R_1$ is H or a halogen atom selected from the group consisting of F, Cl, Br and I, preferably F or Cl;
$R_2$ is H or an halogen atom selected from the group consisting of F, Cl, Br and I, preferably H or Cl;
$R_3$ is a chloromethyl or a fluoromethylthio group; and
$R_4$ is a furoate or a propionate residue.

Preferably, the steroid is fluticasone furoate or propionate or mometasone furoate.

Due to hydrophobic microenvironment created by the carrier particles coated with magnesium stearate, the chemical stability of said kind of active ingredients in the formulation during storage is improved.

In particular very significant results have been obtained with antimuscarinic drugs bearing a carbonate group of general formula (II).

The following example illustrates in detail the invention.

**EXAMPLE**

The investigation was aimed at evaluating the chemical compatibility of active ingredients bearing a group susceptible to hydrolysis with carriers for powders for inhalation.

**Test compound**

The drug used as test compound was (R)-3-[bis-(3-fluoro-phenyl)-methoxycarbonyloxy]-1-(2-oxo-2-thiophen-2-yl-ethyl)-1-azonia-bicyclo[2.2.2]octane; chloride.

The drug and its synthesis are disclosed in WO 2010/015324.

It was micronized before use leading to a particle size of d(v, 0.1)=0.7 μm, d(v, 0.5)=2.7 μm, d(v, 0.9)=9.2 μm.

**Excipients:**

Alpha-lactose monohydrate commercially available as Capsulac® 60 (Meggle) was used.

The fraction of interest, 212-355 micron was obtained by sieving.

Magnesium stearate of vegetable origin was used having a starting particle size with a MMD of less than 10 micron.

**Formulations**

Dry powder formulations were prepared comprising as a carrier either particles of alpha-lactose monohydrate 212-355 micron alone (coarse) or coated with magnesium stearate (coated).

Said coated particles were obtained by mixing alpha-lactose monohydrate with 1% magnesium stearate in a Turbula mixer for 4 hours.

The unit formulae are reported in Table 1

**Table 1**

| Strength | Coarse 20 μg/20 mg | Coated 20 μg/20 mg |
|---|---|---|
| Lactose 212-355 | 19.98 mg | 19.77 |
| Magnesium stearate 1% | - | 0.21 |
| Test compound | 0.020 mg | 0.02 mg |
| Total | 20 mg | 20 mg |

Samples of the manufactured mixtures were filled in glass vials and stored under stressing conditions (90°C, 60% relative humidity and 90°C, 75% relative humidity) in comparison to a sample stored under long tem conditions (25°C, 60% relative humidity).

The percentage amount of residual test compound and of the degradation product due to hydrolysis [(R)-1-hydroxy-(2-oxo-2-thiophen-2-yl-ethyl)-1-azonia-bicyclo[2.2.2]octane; chloride quoted as CHF 5551.02] was determined in the two formulations by ultra performance liquid chromatography (UPLC) in comparison to the active ingredient without the carrier (API).

The results are reported in Tables 2 and 3.

The variation of CHF 5551.2 content with the time is also plotted in Figure 2.

**Table 2**

| Test compound values (%) | Carrier | - | Coarse | Coarse | Coated | Coated |
|---|---|---|---|---|---|---|
| | Temperature (°C) | 25 | 90 | 90 | 90 | 90 |
| | Rel. humidity (%) | 60 | 60 | 75 | 60 | 75 |
| Check point no. | Time (days) | | | | | |
| 0 | 0 | 100.0 | 100.0 | 100.0 | 100.0 | 100.0 |
| 1 | 2 | 99.9 | 99.9 | 100.0 | 100.0 | 100.0 |
| 2 | 4 | 99.9 | 99.8 | 99.8 | 99.98 | 99.9 |
| 3 | 7 | 99.9 | 99.7 | 99.5 | 99.8 | 99.6 |
| 4 | 9 | 99.9 | 99.6 | 99.5 | 99.8 | 99.6 |
| 5 | 11 | 99.9 | 99.5 | 99.4 | 99.8 | 99.6 |
| 6 | 14 | 100.0 | 99.3 | 99.3 | 99.8 | 99.5 |

**Table 3**

| CHF 5551.02 values (%) | Carrier | - | Coarse | Coarse | Coated | Coated |
|---|---|---|---|---|---|---|
| | Temperature (°C) | 25 | 90 | 90 | 90 | 90 |
| | Rel. humidity (%) | 60 | 60 | 75 | 60 | 75 |
| Check point no. | Time (days) | | | | | |

(continued)

| CHF 5551.02 values (%) | Carrier | - | Coarse | Coarse | Coated | Coated |
|---|---|---|---|---|---|---|
| | Temperature (°C) | 25 | 90 | 90 | 90 | 90 |
| | Rel. humidity (%) | 60 | 60 | 75 | 60 | 75 |
| 0 | 0 | 0.08 | 0.08 | 0.08 | 0.07 | 0.07 |
| 1 | 2 | 0.20 | 0.27 | 0.25 | 0.24 | 0.26 |
| 2 | 4 | 0.29 | 0.38 | 0.43 | 0.39 | 0.37 |
| 3 | 7 | 0.24 | 0.56 | 0.66 | 0.44 | 0.63 |
| 4 | 9 | 0.28 | 0.56 | 0.74 | 0.47 | 0.66 |
| 5 | 11 | 0.29 | 0.74 | 0.81 | 0.51 | 0.66 |
| 6 | 14 | 0.23 | 0.90 | 0.87 | 0.52 | 0.80 |

The results indicate that the amount of the degradation product due to hydrolysis increases moving from a carrier made of only alpha-lactose monohydrate to the coated particles.

## Claims

1. Use of magnesium stearate in powder formulations for inhalation comprising carrier particles of alpha-lactose monohydrate and an active ingredient bearing a group susceptible to hydrolysis selected from the group consisting of carbonate, carbamate and ester, to inhibit or reduce chemical degradation of said active ingredient, wherein the carrier particles have the mass diameter comprised between 80 and 500 micron and wherein magnesium stearate is present in an amount comprised between 0.05 and 1.5% based on the total weight of the carrier and coats the surface of the carrier particles to an extent higher than 60%.

2. The use according to claim 1, wherein the amount is of from 0.1 to 1.0% w/w.

3. The use according to claims 1 or 2, wherein the mass diameter is comprised between 210 and 355 micron.

4. The use according to any one of the preceding claims, wherein magnesium stearate coats the surface of the carrier particles to an extent higher than 70%.

5. The use according to claim 4, wherein magnesium stearate coats the surface of the carrier particles to an extent of at least 80%.

6. The use according to any one of the preceding claims, wherein the active ingredient is selected from the classes consisting of antimuscarinic drugs, phosphodiesterase-4 inhibitors, and steroids for inhalation.

## Patentansprüche

1. Verwendung von Magnesiumstearat in Pulverformulierungen zur Inhalation, die Trägerpartikel von alpha-Lactosemonohydrat und einen aktiven Inhaltsstoff, der eine Gruppe trägt, die hydrolyseempfindlich ist, ausgewählt aus der Gruppe, bestehend aus Carbonat, Carbamat und Ester, umfassen, um den chemischen Abbau des aktiven Inhaltsstoffes zu inhibieren oder zu reduzieren, wobei die Trägerpartikel den Massendurchmesser, der zwischen 80 und 500 Mikrometer liegt, besitzen und wobei das Magnesiumstearat in einer Menge, die zwischen 0,05 und 1,5%, basierend auf dem Gesamtgewicht des Trägers, liegt, vorliegt und die Oberfläche der Trägerpartikel bis zu einem Ausmaß größer als 60% beschichtet.

2. Verwendung gemäß Anspruch 1, wobei die Menge von 0,1 bis 1,0% G/G beträgt.

3. Verwendung gemäß den Ansprüchen 1 oder 2, wobei der Massendurchmesser zwischen 210 und 355 Mikrometer

liegt.

4. Verwendung gemäß einem der vorangehenden Ansprüche, wobei das Magnesiumstearat die Oberfläche der Trägerpartikel bis zu einem Ausmaß größer als 70% beschichtet.

5. Verwendung gemäß Anspruch 4, wobei das Magnesiumstearat die Oberfläche der Trägerpartikel bis zu einem Ausmaß von wenigstens 80% beschichtet.

6. Verwendung gemäß einem der vorangehenden Ansprüche, wobei der aktive Inhaltsstoff ausgewählt ist aus den Klassen, bestehend aus antimuskarinischen Arzneimitteln, Phosphodiesterase-4-Inhibitoren und Steroiden zur Inhalation.

**Revendications**

1. Utilisation de stéarate de magnésium dans des formulations pulvérulentes à des fins d'inhalation comprenant des particules de support de monohydrate d'alpha-lactose et un ingrédient actif portant un groupe sensible à l'hydrolyse choisi parmi le groupe constitué par un carbonate, un carbamate et un ester, destiné à inhiber ou à réduire la dégradation chimique dudit ingrédient actif ; dans laquelle les particules de support possèdent un diamètre de masse compris entre 80 et 500 microns ; et dans laquelle le stéarate de magnésium est présent en une quantité comprise entre 0,05 et 1,5 %, basé sur le poids total du support, et recouvre la surface des particules de support jusqu'à concurrence d'une valeur supérieure à 60 %.

2. Utilisation selon la revendication 1, dans laquelle la quantité s'élève de 0,1 à 1,0 % en poids/poids.

3. Utilisation selon la revendication 1 ou 2, dans laquelle le diamètre de masse est compris entre 210 et 355 microns.

4. Utilisation selon l'une quelconque des revendications précédentes, dans laquelle le stéarate de magnésium recouvre la surface des particules de support jusqu'à concurrence d'une valeur supérieure à 70 %.

5. Utilisation selon la revendication 4, dans laquelle le stéarate de magnésium recouvre la surface des particules de support jusqu'à concurrence d'une valeur au moins égale à 80 %.

6. Utilisation selon l'une quelconque des revendications précédentes, dans laquelle l'ingrédient actif est choisi parmi les classes constituées par des médicaments antimuscariniques, des inhibiteurs des phosphodiestérases de type 4 et des stéroïdes destinés à l'inhalation.

Figure 1

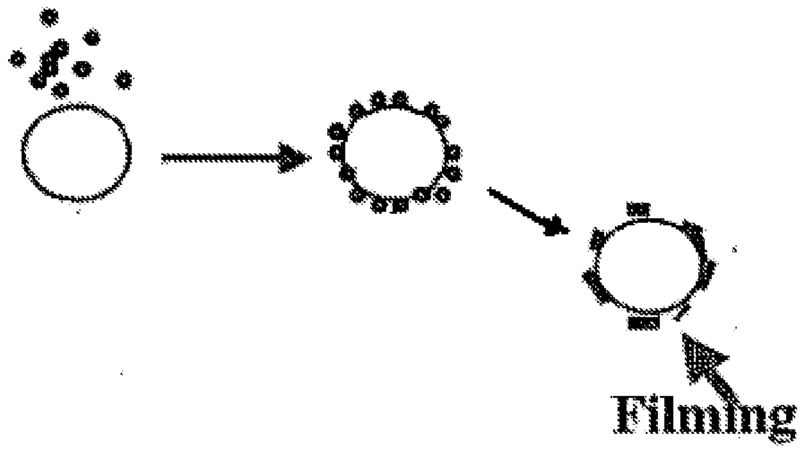

# Figure 2

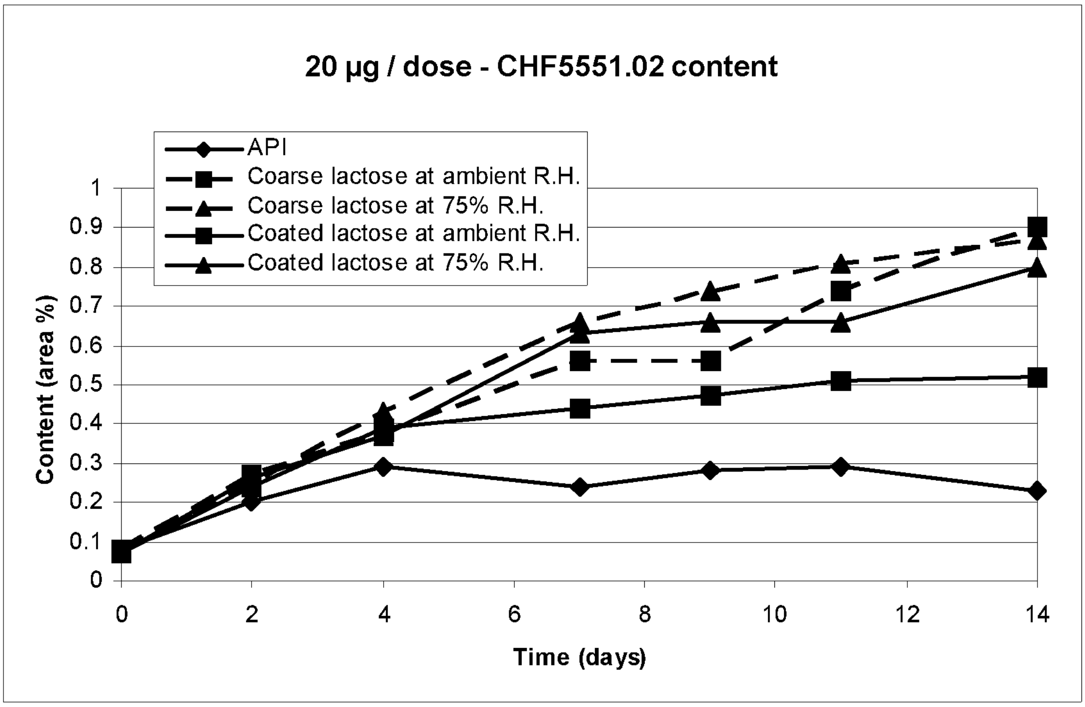
20 µg / dose - CHF5551.02 content
API
Coarse lactose at ambient R.H.
Coarse lactose at 75% R.H.
Coated lactose at ambient R.H.
Coated lactose at 75% R.H.
Content (area %)
Time (days)
0
0.1
0.2
0.3
0.4
0.5
0.6
0.7
0.8
0.9
1
0
2
4
6
8
10
12
14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6528096 B
- WO 0028979 A
- WO 2005004845 A
- WO 0178695 A
- WO 0178693 A
- EP 10158951 A
- WO 2010015324 A
- WO 2009018909 A


### Non-patent literature cited in the description


- **COLOMBO I et al.** *Il Farmaco,* 1984, vol. 39 (10), 338